# EUROPEAN PATENT SPECIFICATION

(11) **EP 1 113 864 B1**
(45) Date of publication and mention of the grant of the patent: **30.06.2004**
(21) Application number: 99939449.7
(22) Date of filing: 03.08.1999
(51) Int. Cl.: B01F 17/00

(54) **PESTICIDE FORMULATIONS CONTAINING ETHOXYLATED AMINE NEUTRALIZED AROMATIC SULFONIC ACID SURFACTANTS**
PESTIZIDFORMULIERUNG ENTHALTEND TENSIDE ERHALTEN DURCH NEUTRALISIERUNG EINER AROMATISCHEN SULFONSÄURE MITTELS EINER ETHOXLIERTEN AMINE
FORMULATIONS PESTICIDES CONTENANT DES TENSIOACTIFS D'AMINE ETHOXYLEE NEUTRALISES PAR UN ACIDE SULFONIQUE AROMATIQUE

(30) Priority: 05.08.1998 US 128950
(43) Date of publication of application: 11.07.2001
(73) Proprietor: Syngenta Participations AG, 4058 Basel (CH)
(72) Inventor: SHANNON, Tammy, Tyler, Winfield, WV 25213 (US); MOORE, Carolyn, Estep, Kernersville, NC 27284 (US); CHOW, Victor, Shui-Chiu, Jamestown, NC 27282 (US)
(74) Representative: Bastian, Werner Maria
(86) International application number: PCT/EP1999/005628
(87) International publication number: WO 2000/007709

(56) References cited:
- EP-A- 0 074 329
- FR-A- 2 297 843

## Description

### FIELD OF THE INVENTION

The present invention relates to surfactants useful in life science formulations. More particularly, the invention relates to pesticide formulations containing surfactants of ethoxylated amine neutralized by aromatic sulfonic acid.

### BACKGROUND OF THE INVENTION

Alkylphenol ethoxylates (APEs) are a class of common industrial surfactants which are widely used in pesticide formulations. However, formulations containing APEs do not always provide the most desirable combination of design specifications, e.g. product efficacy, working parameters and cost. Traditional non-APE surfactant systems have not been readily adaptable substitutes for APE surfactants. For example, depending on the formulation requirements, calcium dodecylbenzenesulfonate used in conjunction with fatty acid ethoxylates has proven to be an unacceptable APE substitute because of poor performance within one or more design parameters such as emulsion stability, acute toxicity, temporal and thermal stability, chemical and physical stability; solution, suspension or dilution dynamics, and viscosity and suspension stabilization. Phosphate esters, which are non-APE surfactants found in many types of formulations, suffer from long term stability problems due to undesirable transesterification and saponification rections involving the mono-ester, di-ester, and free acid components of the surfactant mixture. The inability of industry to adapt existing technology to improve upon characteristics of surfactant systems containing APEs has prompted the development of entirely new line non-APE surfactant substitutes. The additional challenge faced by the scientific community has been to develop new non-APE substitutes which can be easily made from readily available and cost effective raw materials. Accordingly, there continues to be a need for other non-APE surfactant substitutes.
FR-A-2 297 843 discloses the salt of an alkylaryl sulphonic acid and an organic base containing at least one -(C₂H₄O)ₙ-H group, wherein n is a number larger than 1 and smaller than 40 and the base contains no alkyl group of 6 or more carbon atoms as emulsifiers.
EP-A-0 074 329 discloses concentrated aqueous solutions or suspensions of pesticides comprising aromatic sulphonates with either alkali metal, ammonium, primary, secondary or tertiary ammonium, hydroxyalkylammonium or morpholinium cations.

### SUMMARY OF THE INVENTION

It has now been found that ethoxylated amine neutralized aromatic sulfonic acids are suitable as non-APE surfactant substitutes. The surfactants of the instant invention may be in the form of a surfactant salt compound, or composition containing one or more of the surfactant compounds or salts. In one embodiment of the invention, the compositions containing the instant non-APE surfactants do not contain or are substantially free of alkylphenol ethoxylates (APEs). The surfactants of the instant invention are obtained from combining the appropriate aromatic sulfonic acid with the appropriate ethoxylated amine.

### DETAILED DESCRIPTION OF THE INVENTION

One aspect of the invention is a pesticide formulation comprising at least one pesticide and the salt compound of formula (I):

[(H-B)⁺]ₙ Aⁿ⁻ (I),

wherein Aⁿ⁻ is the conjugate base of the acid H-A, wherein H-A is an aromatic sulfonic acid; and (H-B)⁺ is the conjugate acid of the base B, wherein B is an ethoxylated amine, and n is the number of sulfonate anion groups or sulfonic acid groups on the conjugate base or acid respectively and with the proviso that HA and Aⁿ⁻ may not contain a phenolic moiety. A "phenolic moiety" is defined as any phenyl ring containing at least one -OH group or any group developed by replacing the hydrogen of the -OH group with another organic or metal radical. The invention disclosed herein also includes a method for the preparation of a salt compound of formula (I). The invention also includes a method of protecting crops.

A preferred aspect of the invention is a pesticide formulation comprising the salt compound having the formula [(H-B)⁺]ₙ Aⁿ⁻ wherein Aⁿ⁻ is the anion of the formula: 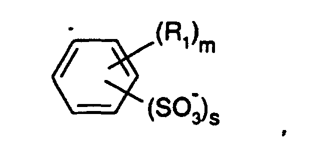 wherein each R₁ is independently selected from the group consisting of C₈-C₂₂ alkyl, and C₈-C₂₂ alkylaryl, m is a number from 1 to 5, n is a number from 1 to 3, and s is a number from 1 to 3, and (H-B)⁺ is the cation of the formula: 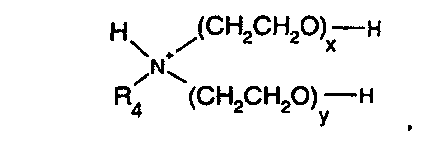 wherein R₄ is selected from the group consisting of C₇-C₁₉ alkyl and C₇C₁₉ alkenyl-CH₂-, and x + y is a number from 2 to 15. A more preferred embodiment of the pesticide formulation is the salt compound of formula (H-B)⁺A⁻ , wherein A⁻, is the anion of the formula: 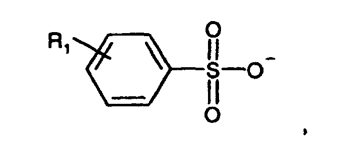 wherein R₁ is C₈-C₂₂ alkyl and (H-B)⁺ is the cation of the formula: 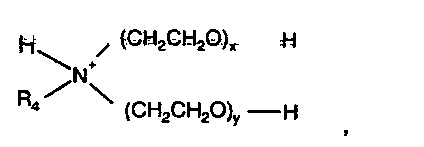 wherein R₄ is defined above (preferably n-octadecyl, n-hexadecyl and cis-9-octadecenyl); and x + y is a number from about 7 to 9 (preferably 8). Another more preferred embodiment of the invention is a pesticide formulation comprising the salt compound wherein A⁻ is an anion having the formula: 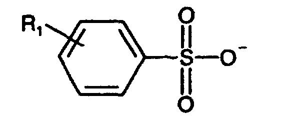 wherein R, is n-dodecyl group.

The invention also is the compound or salt produced from the process of neutralizing an aromatic sulfonic acid of formula HA with an ethoxylated amine of formula B. The ammoniumsulfonate salt compounds or the product from the process of combining an aromatic sulfonic acid with an ethoxylated amine (to the extent there is a difference) are both features of the instantly disclosed invention. The scope of the invention disclosed herein should not be construed to be limited by any particular chemical theory relating to the complexation, equilibration, reaction or acid-base chemistry of the components used to make the surfactants or other ingredients used to make other ultimately useful formulations (e.g. pesticide formulations). In this regard, another aspect of the invention is the surfactant composition comprising one or more aromatic sulfonic acids and one or more ethoxylated amines wherein the constituent components may or may not have interacted chemically so as to result in a change in form of the components. The invention encompasses the static composition of the appropriate components admixed together as well as the chemically integrated surfactant composition comprising at least one aromatic sulfonic acid and at least one ethoxylated amine. "Static composition" denotes the composition composed of components wherein the components have not substantially changed by virtue of their combination with other composition components. "Chemically integrated composition" means a composition that results from the natural equilibration, complexation, dissociation or other chemical transformation if any that may occur after combination of the components and prior to ultimate use of the surfactant in a pesticide formulation. Therefore, the "chemically integrated composition" of the instant invention by definition encompasses the situation where there is a "static composition" as well as any resultant composition occurring at any point in time between initial creation and ultimate use in the field of products containing the surfactant. In other words, the disclosed invention is not limited to a static composition of chemically unaltered constituent components.

Another aspect of the invention is the surfactant composition produced as a whole from the combination of the aromatic sulfonic acid and the ethoxylated amine base to the extent it is composed of products other than ammoniumsulfonate salt compounds. Such a composition may contain chemically unaltered starting materials as well as other reaction products or by-products from reaction, equilibration, dissociation or complexation of the components in the composition.

The invention also includes the method for obtaining a surfactant which is useful as an APE substitute. In addition to the surfactant product, the method of obtaining or making any of the surfactants is herein disclosed as part of the invention.

The ethoxylated amines and the aromatic sulfonic acids used to make the surfactants of the invention are preferably those compounds that are readily available and inexpensive. However, cost of materials is only one factor in selecting the ethoxylated amines and the aromatic acids used as starting materials. After performing a routine cost-benefit analysis and in view of other design parameters it may become apparent that more expensive and less readily available starting materials may be preferred.

The aromatic sulfonic acids used in the instant invention may have one or more sulfonic acid groups and one or more groups attached to the aromatic moiety therein. The aromatic sulfonic acids may generally be defined by formula II: 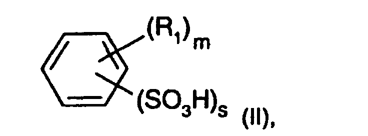 wherein each R₁, m, n and s are defined as above.

The preferred starting materials for making the surfactants of the instant invention are the compounds defined by formula IIa: 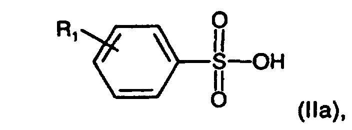 wherein R₁ is defined as above.

The preferred ethoxylated amines useful for making the surfactants of the instant invention are one or more of the compounds defined by formula IV: 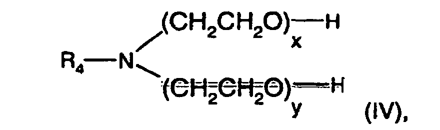 wherein R₄, x and y are defined as above.
Additional preferred aspects of the invention are where R₄ is selected from the group consisting of n-octadecyl, n-hexadecyl and cis-9-octadecenyl, and x + y is a number from 5-10. Another preferred aspect of the invention is where the average x + y is a number from about 7 to 9 (more preferably 8).

Another aspect of the invention is the composition comprising the formulation of the surfactants disclosed above in formulations that contain one or more other active ingredients. The invention is a pesticide formulation which contains the presently disclosed non-APE containing surfactants. The surfactants presently disclosed are considered to have general applicability as APE substitutes, and therefore would be expected to be useful in many other known formulations. Although the compounds and compositions are referred to as "surfactants" in the instant application, it is expected that they will also have other nonsurfactant properties that may be useful independently of any inherent surfactant properties. Depending on the application of the instant invention, it may result in increased bioefficacy and/or reduced toxicity and irritation.

Another aspect of the invention is the composition comprising the formulation of the surfactants disclosed above in formulations that contain one or more herbicides and one or more safeners (antidote). When applying herbicides, the cultivated plants may also suffer severe damage owing to factors that include the concentration of the herbicide and the mode of application, the cultivated plant itself, the nature of the soil, and the climatic conditions such as exposure. A preferred embodiment of the invention is for example the formulation of s-metolachlor and benoxacor, or s-metolachlor, atrazine and benoxacor each in combination with the surfactants of the instant invention.

The surfactants are prepared by mixing the aromatic sulfonic acid of formula II with the ethoxylated amine of formula IV while controlling pH. The desired pH is maintained by precisely regulating the ratio of the acid and base components in the composition. For example, the appropriate acid base ratio and desired pH can be achieve according to the following procedure: 1) A known weight of the aromatic sulfonic acid is dissolved in a 50/50 solution of isopropanol and water. 2) A tared amount of ethoxylated amine is slowly added to the aromatic sulfonic acid with constant stirring using a magnetic stirrer while pH is monitored by use of a pH meter fitted with a silver chloride electrode. 3) When the desired pH is attained, the amount of required ethoxylated amine is measured. 4) The ratio of aromatic sulfonic acid: ethoxylated amine (acid: base) is determined and the surfactant is prepared by mixing the appropriate amounts of the two components with stirring. For many of the pesticide formulations described herein the preferred acid : base ratio is approximately 35:65 (weight ratio). The acidity or basicity of the constituent components may vary depending on the supplier of the materials or the particular batch, therefore the pH is the controlling factor in preparing the compounds, compositions and formulations of the instant invention. The pKₐ and pK_{b} for the aromatic sulfonic acid and the ethoxylated amine respectively may be varied to some extent by manipulating the type and degree of substitution for the compounds defined by formula II and IV. Therefore, the selection of the particular acid or base used will also effect the acid : base ratio used to make the surfactants as well as the desired pH. A preferred pH range for the surfactant is a pH from approximately 3-7, a more preferred pH range is from approximately 4 to 6, and an ultimately preferred pH range is from approximately 5-6. It is less desirable to adjust pH after the sulfonic acid and amine base components are mixed by the further addition of other acids or bases typically used to raise or lower pH because even minor amounts of additional salts can make a large difference in the observed properties of the product surfactant. It is also less desirable to have additional process steps or to have the added cost associated with purchasing, handling, storage and disposal of additional chemicals.

The emulsion stability of dodecylbenzenesulfonic acid neutralized with an ethoxylated tallow amine is typically greatest when the "average number of ethylene oxide units" (EO) on the tallow amine is 8 (i.e. 8EO). "Average" is defined as the arithmetic mean of a set of real numbers (in this case the number of ethylene oxide units in the ethoxylated amines used to make the surfactant). A preferred feature of the invention is where there is a continuous and symmetrical bell curve population distribution around 8EO. It is also desirable that there be low dispersion preferably within one standard deviation (σ) of the mean (average EO). A decrease in emulsification ability is observed when either 7EO or 9EO tallow amine was used to make the surfactant.

Some commercial suppliers and product names for the ethoxylated amines (i.e. amine ethoxylates) are:

| **amine ethoxylate** | **Supplier *** | **Product Name** | **Average Number of EO** |
|---|---|---|---|
| Tallow amines | Witco | Witcamine TAM-XO (x = average number of EO) | 2, 4, 4.5, 5, 6, 7, 8, 9, 10 and 15 |
| Coco amines | Witco | Varonic K-2XX (XX = average number of EO) | 02, 05, 10 and 15 |
| | Stephan | Toximul TA-X (x = average number of EO) | 2, 4, 4.5, 5, 6, 7, 8, 9, 10 and 15 |
| Oleyl amine | Witco | Varonic Q-202 | 2 |

| | | | |
|---|---|---|---|
| * Addresses: Witco Corporation, 5777 Frantz Road, P.O. Box 646, Dublin, Ohio 43017. Stephan Company, Northfield. Illinois 60093. | | | |

One preferred aspect of the invention includes the combination of the surfactant compositions herein with a liquid pesticide compositions so as to obtain an emulsifiable concentrate formulation which can be directly mixed with water or other aqueous solution to give an aqueous pesticide formulation without special mixing procedures.

By "alkylaryl" is meant an aryl group substituted by one or more alkyl groups, wherein the "aryl" may be either a non-heteroaromatic ring system or heteroaromatic ring system.

The following examples illustrate further some of the aspects of the invention but are not intended to limit its scope. Where not otherwise specified throughout this specification and claims, temperatures are given in degrees centigrade.

### EXAMPLE 1

An oil-based suspension concentrate containing solid glyphosate as active ingredient was prepared according to the following formulation:
36.8% by weight solid glyphosate,
15.0% by weight linear dodecylbenzenesulfonic acid neutralized with tallow amine (8EO),
0.1 % by weight dimethyl polysiloxane as an antifoam agent
1.0% by weight fumed silicate as thickener, and a petroleum hydrocarbon solvent to make up 100%.
Other glyphosate salts may also be used in the invention, such as glyphosate-isopropylammonium, glyphosate-sesquisodium, or glyphosate-trimesium.
Other glyphosate salts may also be used in the invention, such as glyphosate-isopropylammonium, glyphosate-sesquisodium, or glyphosate-trimesium.

### EXAMPLE 2

An aqueous suspension concentrate containing solid atrazine as active ingredient was prepared according to the following formulation:
43.5% by weight atrazine,
2.0% by weight linear dodecylbenzenesulfonic acid neutralized with tallow amine (8EO),
1.15% by weight polyoxypropylene polyoxyethylene block copolymer,
0.10% by weight silicone antifoam,
0.15% by weight xanthan gum as thickener,
0.10% by weight preservative formaldehyde, and water to make up 100%.

A pregel is prepared by mixing thickener and water under high shear conditions. A slurry is prepared using the atrazine, the linear dodecylbenzenesulfonic acid neutralized with tallow amine (8EO) and water. The slurry is ground to reduce particle size of atrazine. The pregel and remaining formulants are added and mixed until material is uniform.

### EXAMPLE 3

An aqueous suspension concentrate containing solid atrazine as active ingredient was prepared in a manner analogous to that of Example 2 according to the following formulation:
43.5% by weight atrazine technical,
2.0% by weight branched dodecylbenzenesulfonic acid neutralized with coco amine (10EO),
1.15% by weight polyoxypropylene polyoxyethylene block copolymer,
0.10% by weight silicone antifoam agent,
0.15% by weight xanthan gum as thickener,
0.10% by weight preservative formaldehyde, and water to make up 100%.

Viscosity of material = 130cP (Viscosity by Rotational (Brookfield) viscometer, ASTM Method D2196)

### EXAMPLE 4

An aqueous suspension concentrate containing solid atrazine as active ingredient was prepared in a manner analogous to that of Example 2 according to the following formulation:
43.5% by weight atrazine,
2.0% by weight linear dodecylbenzenesulfonic acid neutralized with coco amine (15EO),
1.15% by weight polyoxypropylene polyoxyethylene block copolymer,
0.10% by weight silicone antifoam agent,
0.15% by weight xanthan gum as thickener,
0.10% by weight preservative formaldehyde, and water to make up 100%.
Viscosity of material = 125cP (Viscosity by Rotational (Brookfield) viscometer, ASTM Method D2196)

### EXAMPLE 5

An aqueous suspension concentrate containing solid atrazine as active ingredient was prepared in a manner analogous to that of Example 2 according to the following formulation:
43.5% by weight atrazine technical,
2.0% by weight linear dodecylbenzenesulfonic acid neutralized with tallow amine (9EO), 1.15% by weight polyoxypropylene polyoxyethylene block copolymer,
0.10% by weight silicone antifoam agent,
0.15% by weight xanthan gum as thickener,
0.10% by weight preservative such as formaldehyde and water to make up 100%.
Viscosity of material = 324 cP (Viscosity by Rotational (Brookfield) viscometer, ASTM Method D2196)

Additionally, one or more nonionic surfactant components may be used in the crop protection formulations. Nonionic surfactants are preferably polyglycol ether derivatives of aliphatic alcohols. The nonionic surfactants for example may be ethylenoxy/propylenoxy block polymers, castor oil ethoxylate and tristyarylphenol ethoxylate.

The crop protection compositions may be formulated in a form suitable for the intended application. Types of formulations include for example a flowable (FL) flowable concentrate for seed treatment (FS), wettable powder (WP), wettable dispersible granules (WDG), oil miscible flowable concentrate (OF), suspension concentrate (SC), emulsifiable concentrate (EC), liquid (L), water in oil emulsions (EW), granules (GR) water dispersible powder for slurry treatment (WS) and dry flowable (DF).

Some additional preferred embodiments of the instant invention are contained in Tables 1 and 2 below.

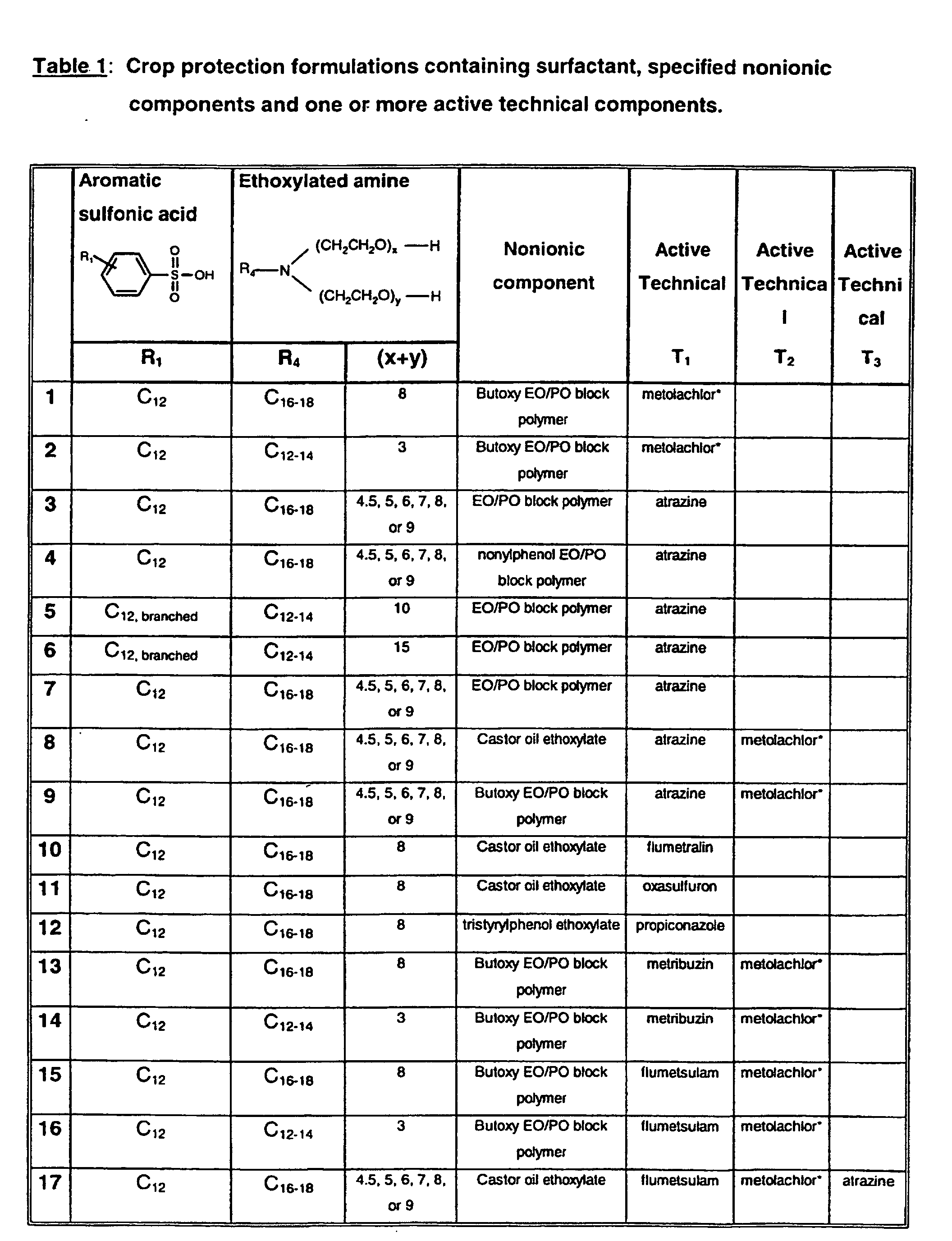

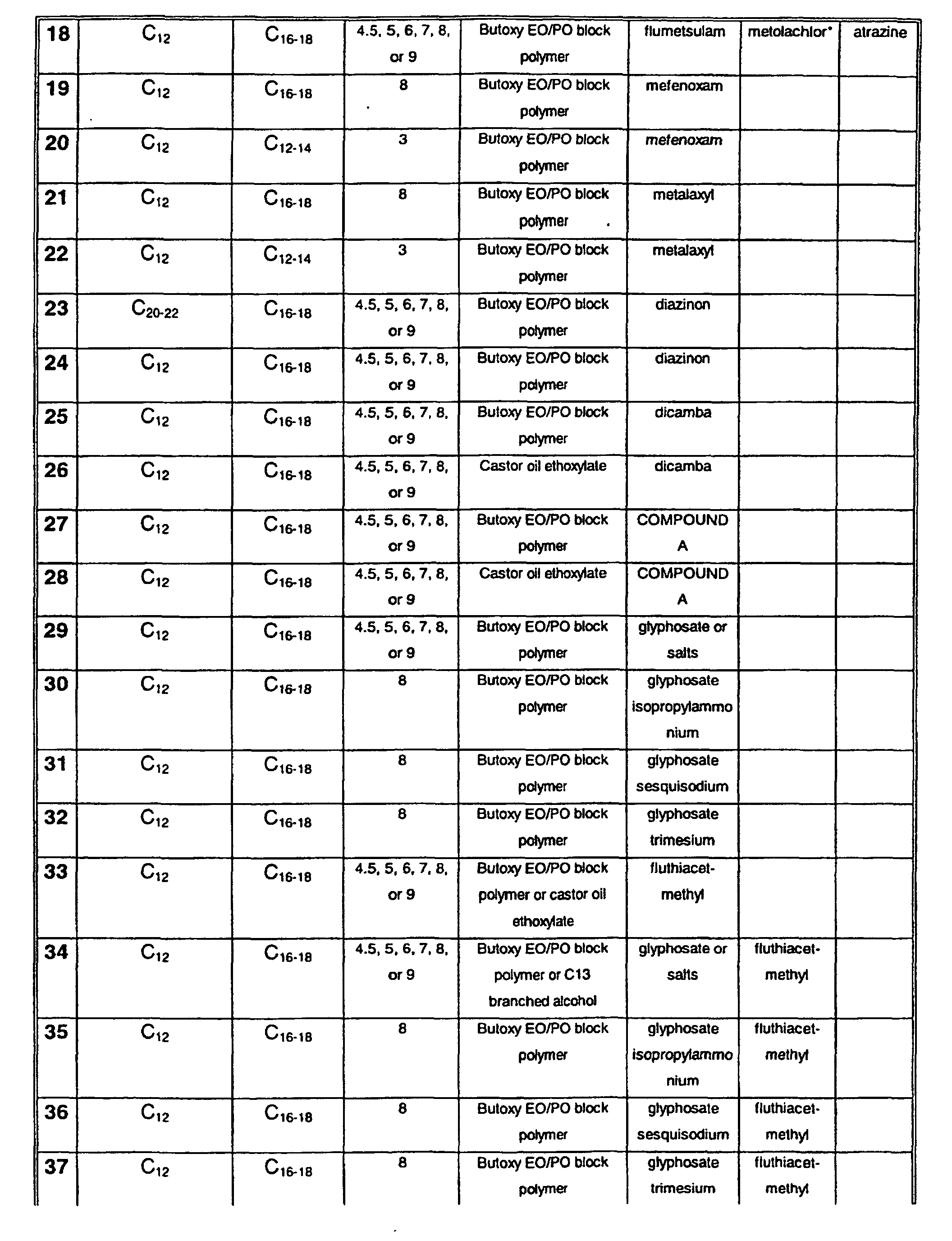

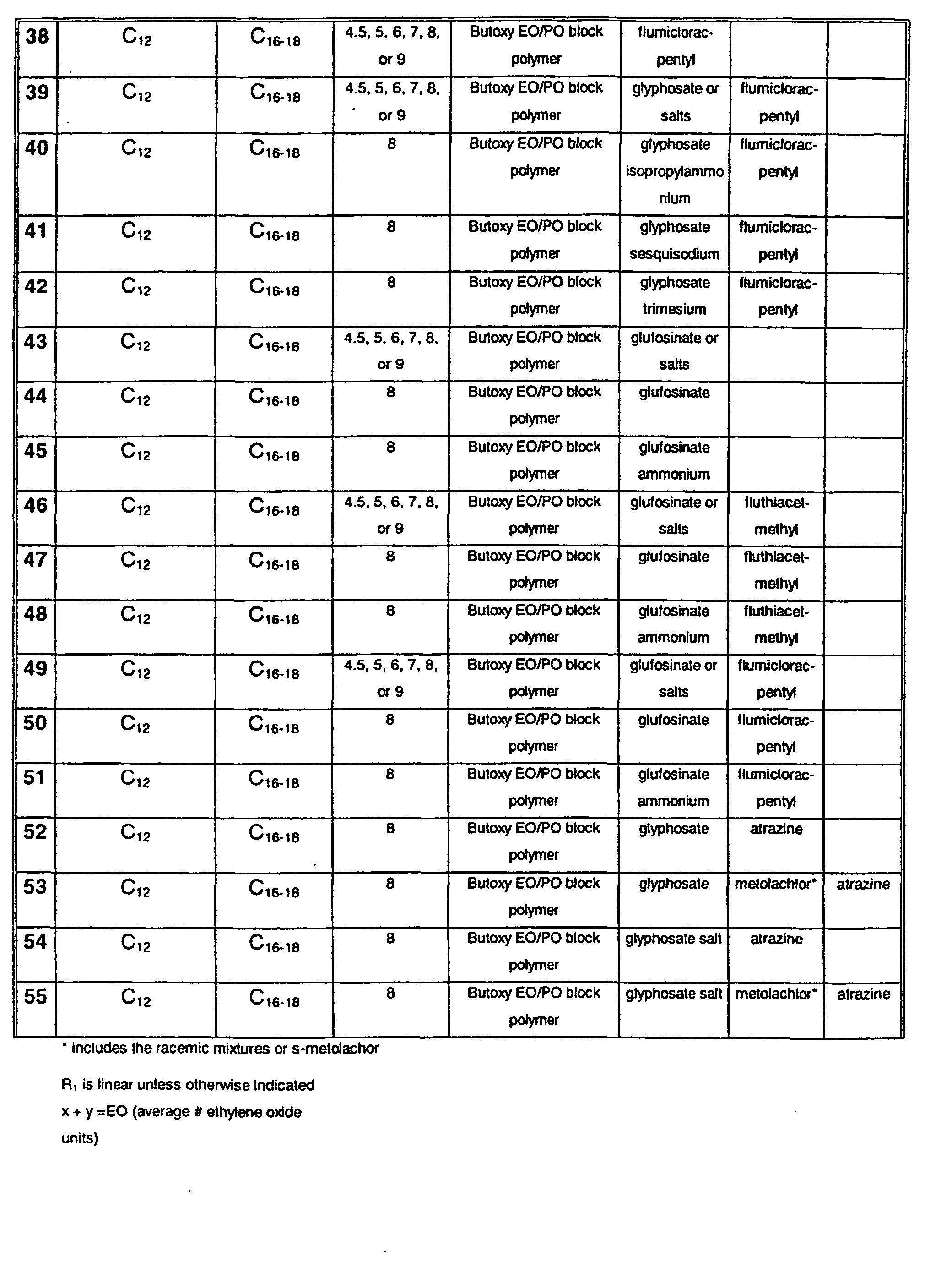

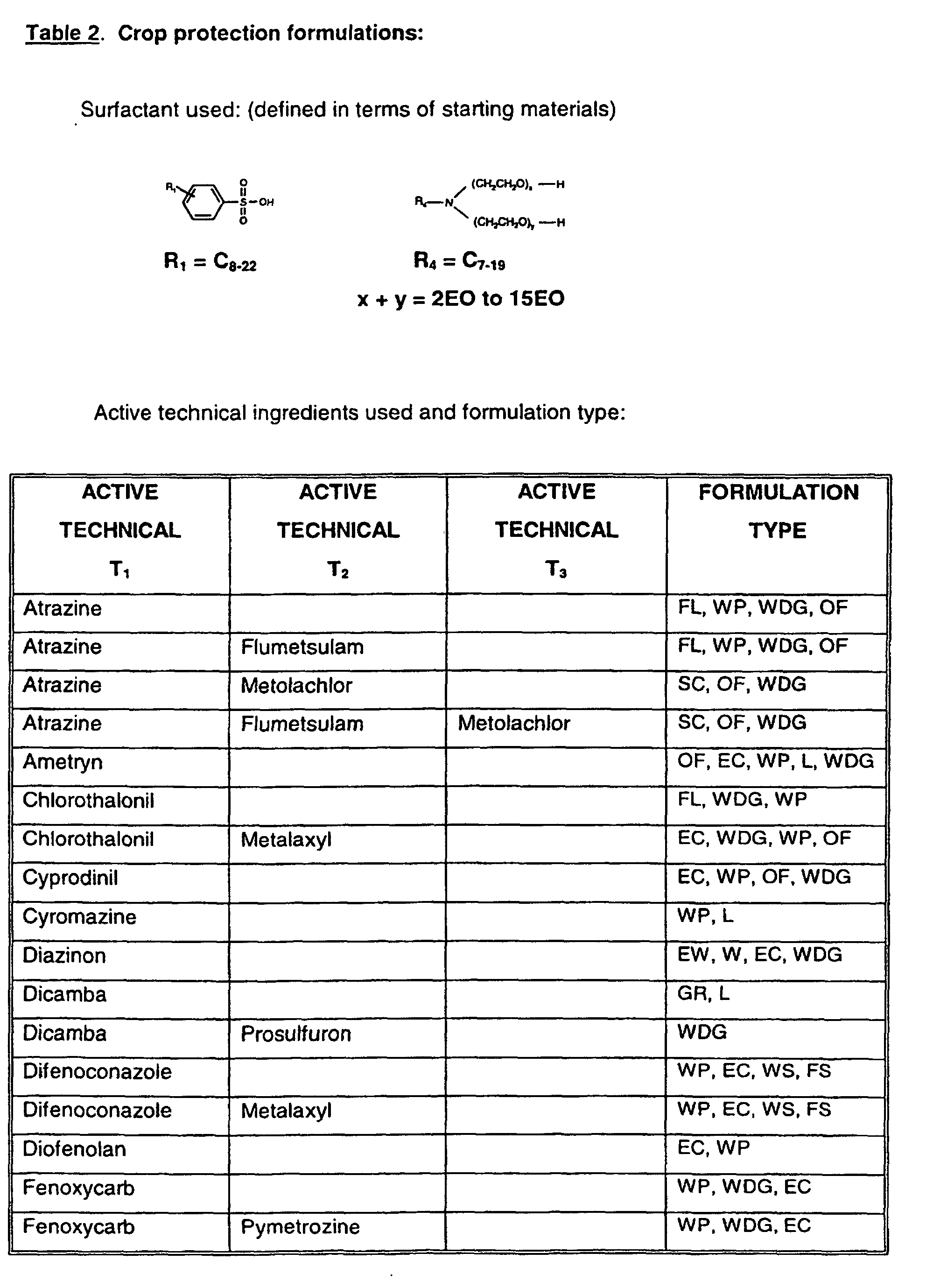

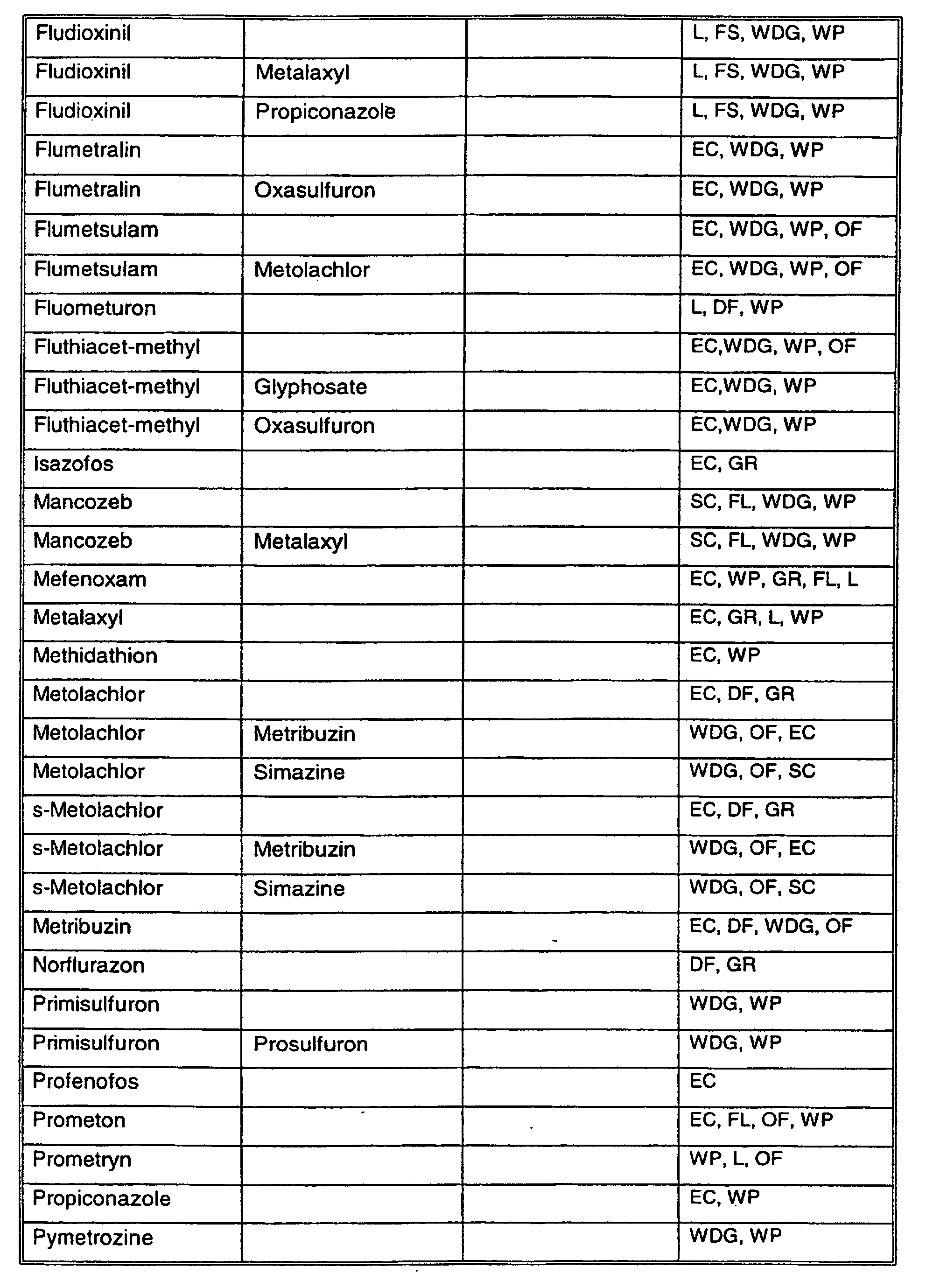

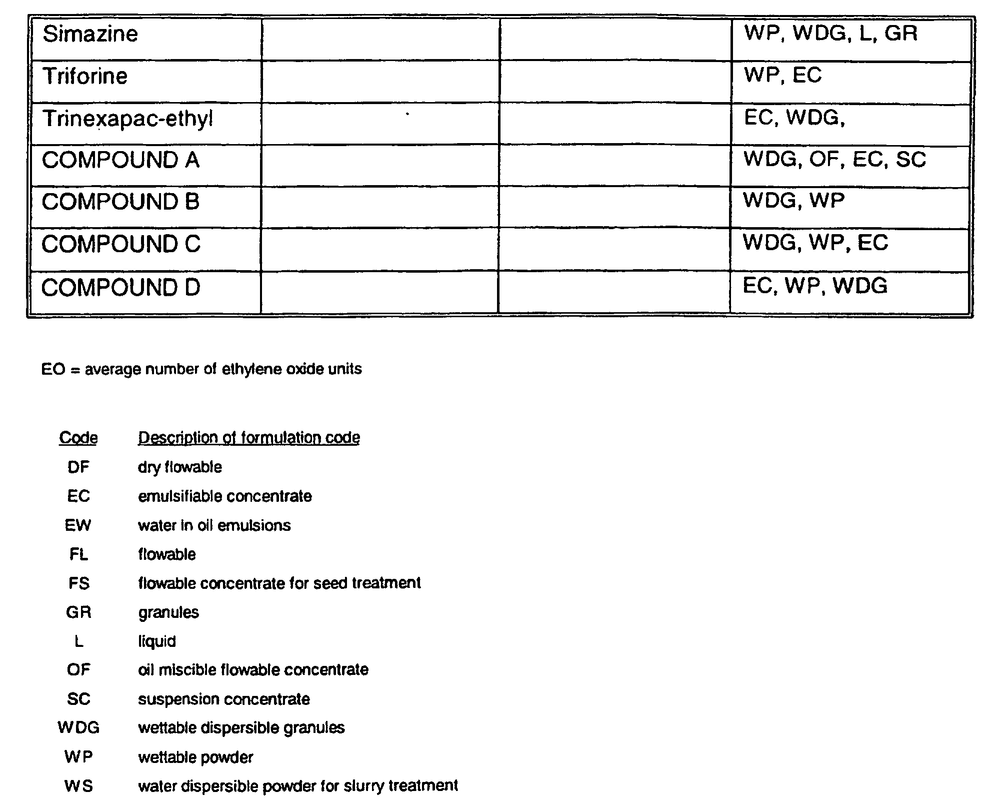

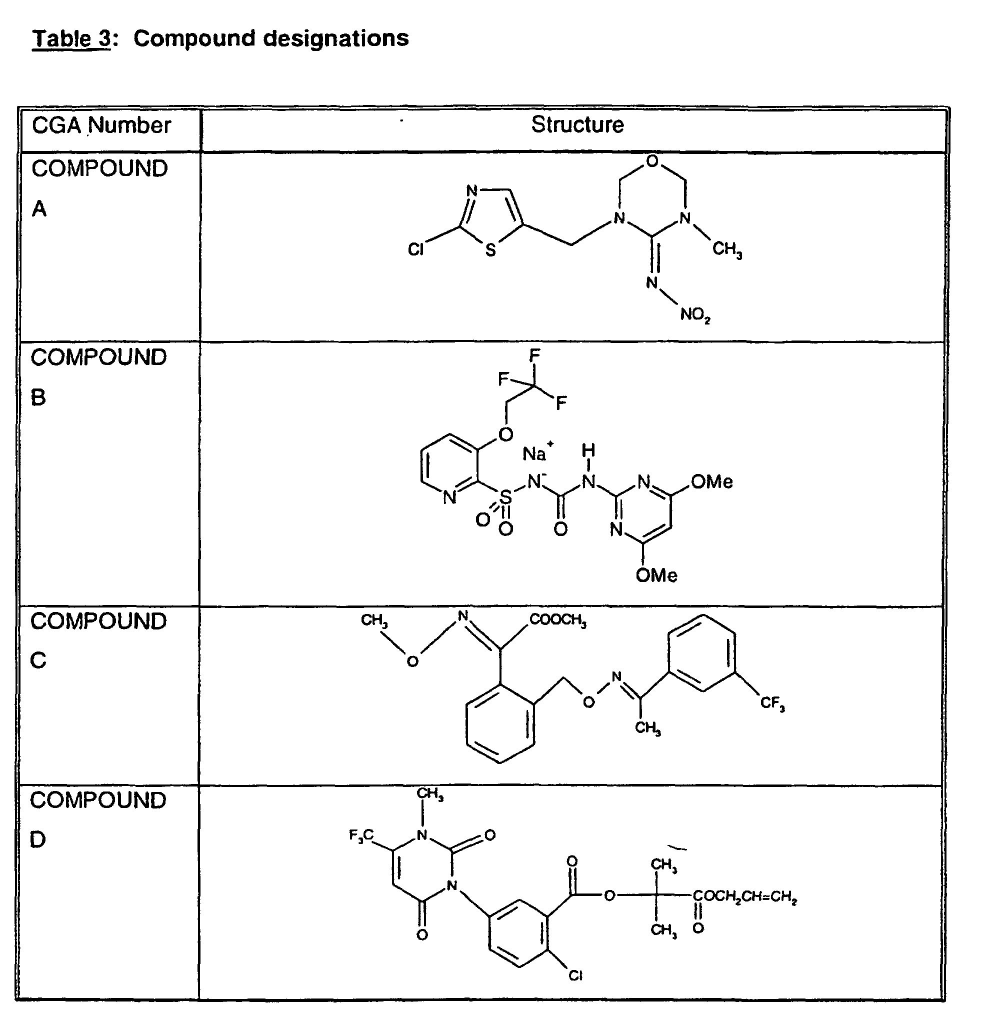

## Claims

1. A pesticide formulation comprising at least one pesticide and a salt compound of the formula [(H-B)⁺]ₙ Aⁿ⁻
wherein Aⁿ⁻ is: 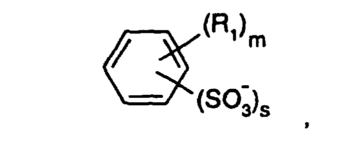 wherein each R₁ is independently selected from the group consisting of C₈-C₂₂ alkyl and C₈-C₂₂ alkylaryl, m is a number from 1 to 5, n is a number from 1 to 3, and s is a number from 1 to 3,
wherein (H-B)⁺ is the cation of the formula: 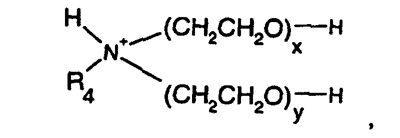 wherein R₄ is selected from the group consisting of C₇-C₁₉ alkyl and C₇-C₁₉ alkenyl-CH₂-, and x + y is a number from 2 to 15.

2. A pesticide formulation according to claim 1, having the formula (H-B)⁺A⁻ , wherein A⁻ is: 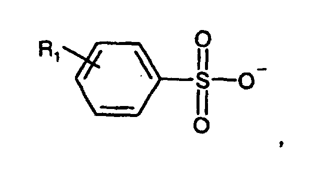 wherein R₁ is C₈-C₂₂ alkyl;
wherein (H-B)⁺ is the cation of the formula: 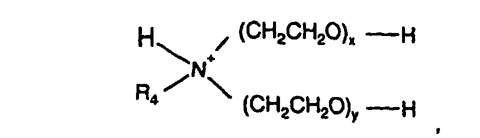 wherein x + y is a number from about 7 to 9, and R₄ is defined above.

3. A pesticide formulation according to claim 2, wherein R₁ is a n-dodecyl group.

4. A pesticide formulation according to claim 2, wherein R₄ is selected from the group consisting of n-octadecyl, n-hexadecyl and cis-9-actadecenyl; and x + y is equal to 8.

5. A method for the preparation of a pesticide formulation according claim 1, **characterised by** comprising the step of neutralizing at least one aromatic sulfonic acid of formula II: 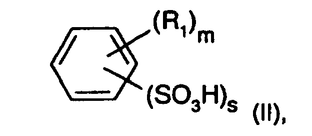 wherein each R₁ is independently selected from the group consisting of C₈-C₂₂ alkyl and C₈-C₂₂ alkylaryl, m is a number from 1 to 5, n is a number from 1 to 3, and s is a number from 1 to 3,
with at least one ethoxylated amine base of formula IV: 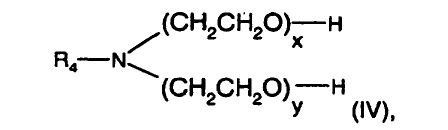 wherein R₄ is selected from the group consisting of C₇-C₁₉ alkyl and C₇C₁₉ alkenyl-CH₂-, and x + y is a number from 2 to 15 inclusive.

6. A method according to claim 5, **characterised in that** said at least one aromatic sulfonic acid is a compound defined by formula IIa: 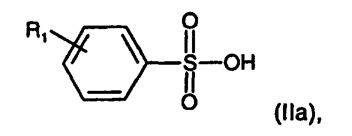 wherein R₁ is C₈-C₂₂ alkyl; and
said at least one ethoxylated amine base represented by the formula IV: 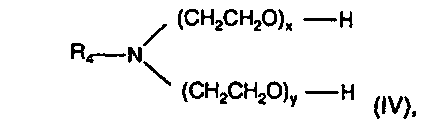 wherein R₄ is C₇-C₁₉ alkyl and x + y is a number from about 7 to 9.

7. A method according to claim 5, **characterised in that** the ratio of the aromatic sulfonic acid and ethoxylated amine base components II and IV in the solution of isopropanol and water is precisely regulated such that the pH range as the controlling factor in preparing the surfactant compound of the formula [(H-B)⁺]ₙ Aⁿ is a pH from 3 to 7.

8. A method according to claim 5, **characterised in that** the ratio of the aromatic sulfonic acid and ethoxylated amine base components II and IV in the solution of isopropanol and water is precisely regulated such that the pH range as the controlling factor in preparing the surfactant compound of the formula [(H-B)⁺]ₙ Aⁿ⁻ is a pH from 4 to 6.

9. A method according to claim 5, **characterised in that** the ratio of the aromatic sulfonic acid and ethoxylated amine base components II and IV in the solution of isopropanol and water is precisely regulated such that the pH range as the controlling factor in preparing the surfactant compound of the formula [(H-B)⁺]ₙ Aⁿ⁻ is a pH from 5 to 6.

10. A pesticide formulation of claim 1, wherein the at least one pesticide is selected from the group consisting of mefenoxam, s-metolachlor, flumetsulam, fluthiacet-methyl, atrazine and glyphosate.

11. A method of protecting crops comprising the step of applying to the locus of the crops in need of protection an agriculturally effective amount of the formulation of claim 1.

12. A method of protecting crops comprising the step of applying to the locus of the crops in need of protection an agriculturally effective amount of the formulation of claim 2.

## Patentansprüche

1. Pestizidformulierung, umfassend zumindest ein Pestizid und eine Salzverbindung der Formel ((H-B)⁺]ₙAⁿ⁻,
worin Aⁿ⁻ 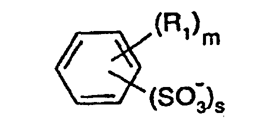 bedeutet, worin jedes R₁ unabhängig ausgewählt ist unter C₈-C₂₂-Alkyl und C₈-C₂₂-Alkylaryl, m eine Zahl von 1 bis 5 bedeutet, n eine Zahl von 1 bis 3 bedeutet, und s eine Zahl von 1 bis 3 bedeutet,
worin (H-B)⁺ das Kation der Formel 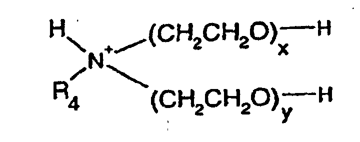 ist, worin R₄ unter C₇-C₁₉-Alkyl und C₇-C₁₉-Alkenyl-CH₂- ausgewählt ist, und x + y eine Zahl von 2 bis 15 bedeutet.

2. Pestizidformulierung gemäß Anspruch 1 mit der Formel (H-B)⁺A⁻,
worin A⁻ 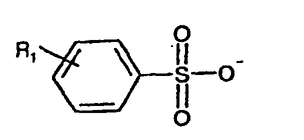 bedeutet, worin R₁ für C₈-C₂₂-Alkyl steht,
worin (H-B)⁺ das Kation der Formel 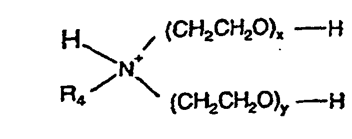 ist, worin x + y eine Zahl von etwa 7 bis 9 ist, und R₄ wie vorstehend definiert ist.

3. Pestizidformulierung gemäß Anspruch 2, worin R₁ eine n-Dodecylgruppe ist.

4. Pestizidformulierung gemäß Anspruch 2, worin R₄ unter n-Octadecyl, n-Hexadecyl und cis-9-Octadecenyl ausgewählt ist, und x und y für 8 steht.

5. Verfahren zur Herstellung einer Pestizidformulierung gemäß Anspruch 1, **gekennzeichnet dadurch, dass** es die Stufe einer Neutralisation zumindest einer aromatischen Sulfonsäure der Formel II 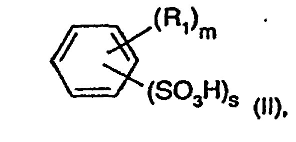 worin jedes R₁ unabhängig ausgewählt ist unter C₈-C₂₂-Alkyl und C₈-C₂₂-Alkylaryl, m eine Zahl von 1 bis 5 bedeutet, n eine Zahl von 1 bis 3 bedeutet, und s eine Zahl von 1 bis 3 bedeutet,
mit zumindest einer ethoxylierten Aminbase der Formel IV 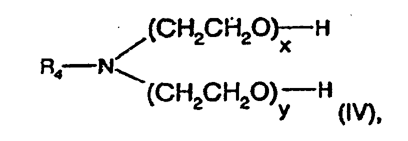 worin R₄ ausgewählt ist unter C₇-C₁₉-Alkyl und C₇-C₁₉-Alkenyl-CH₂-, und x + y eine Zahl von 2 bis 15 einschließlich bedeutet, umfasst.

6. Verfahren gemäß Anspruch 5, **dadurch gekennzeichnet, dass** zumindest eine aromatische Sulfonsäure eine Verbindung der Formel IIa 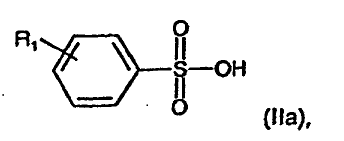 ist, worin R₁ C₈-C₂₂-Alkyl ist, und
die zumindest eine besagte ethoxylierte Aminbase die Formel IV 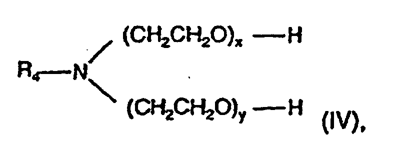 besitzt, worin R₄ C₇-C₁₉-Alkyl ist, und x + y eine Zahl von etwa 7 bis 9 ist.

7. Verfahren gemäß Anspruch 5, **dadurch gekennzeichnet, dass** das Verhältnis der aromatischen Sulfonsäure- und ethoxylierten Aminbasekomponenten II und IV in der Lösung von Isopropanol und Wasser präzise eingestellt wird derart, dass der pH-Bereich als Kontrollfaktor bei der Herstellung der Tensidverbindung der Formel [(H-B)⁺]ₙAⁿ⁻ ein pH von 3 bis 7 ist.

8. Verfahren gemäß Anspruch 5, **dadurch gekennzeichnet, dass** das Verhältnis der aromatischen Sulfonsäure- und ethoxylierten Aminbasekomponenten II und IV in der Lösung von Isopropanol und Wasser präzise derart eingestellt wird, dass der pH-Bereich als Kontrollfaktor bei der Herstellung der Tensidverbindung der Formel [(H-B)⁺]ₙAⁿ⁻ ein pH von 4 bis 6 ist.

9. Verfahren gemäß Anspruch 5, **dadurch gekennzeichnet, dass** das Verhältnis der aromatischen Sulfonsäure- und ethoxylierten Aminbasekomponenten II und IV in der Lösung von Isopropanol und Wasser präzise derart eingestellt wird, dass der pH-Bereich als Kontrollfaktor bei der Herstellung der Tensidverbindung der Formel [(H-B)⁺)ₙAⁿ⁻ ein pH von 5 bis 6 ist.

10. Pestizidformulierung gemäß Anspruch 1, worin zumindest ein Pestizid ausgewählt ist unter Mefenoxam, s-Metolachlor, Flumetsulam, Fluthiacetmethyl, Atrazin und Glyphosat.

11. Verfahren zum Schutz von Nutzpflanzen, umfassend die Stufe des Aufbringens auf den Standort der Nutzpflanzen, die eines Schutzes bedürfen, einer landwirtschaftlich wirksamen Menge der Formulierung gemäß Anspruch 1.

12. Verfahren zum Schutz von Nutzpflanzen, umfassend die Stufe des Aufbringens auf den Standort der Nutzpflanzen, die eines Schutzes bedürfen, einer landwirtschaftlich wirksamen Menge der Formulierung gemäß Anspruch 2.

## Revendications

1. Formulation pesticide comprenant au moins un pesticide et un composé salin ayant la formule [(H-B)⁺]ₙ Aⁿ⁻ dans laquelle Aⁿ⁻ est : 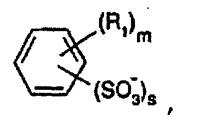 où chaque R₁ est choisi indépendamment dans le groupe constitué par les alkyle en C₈-C₂₂ et les alkylaryle en C₈-C₂₂, m est un nombre compris entre 1 et 5, n est un nombre compris entre 1 et 3, et s est un nombre compris entre 1 et 3,
dans laquelle (H-B)⁺ est le cation ayant la formule : 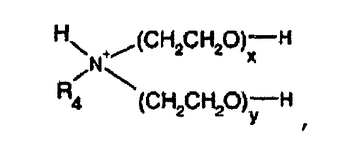 dans laquelle R₄ est choisi dans le groupe constitué par les alkyle en C₇-C₁₉ et les alcényles. en C₇-C₁₉ -CH₂-, et x + y est un nombre compris entre 2 et 15.

2. Formulation pesticide selon la revendication 1, ayant la formule (H-B)⁺ A⁻ dans laquelle A⁻ est : 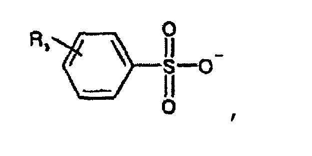 où R₁ est un alkyle en C8-C22 ;
dans laquelle (H-B)⁺ est le cation ayant la formule : 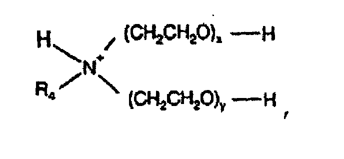 dans laquelle x + y est un nombre compris entre environ 7 et 9, et R₄ est défini ci-dessus.

3. Formulation pesticide selon la revendication 2, dans laquelle R₁ est un groupe n-dodécyle.

4. Formulation pesticide selon la revendication 2, dans laquelle R₄ est choisi dans le groupe constitué par le n-octadécyle, le n-hexadécyle et le cis-9-octadécényle ; et x + y est égal à 8.

5. Procédé de préparation d'une formulation pesticide selon la revendication 1, **caractérisé en ce qu'**il comprend l'étape consistant à neutraliser au moins un acide sulfonique aromatique de formule II : 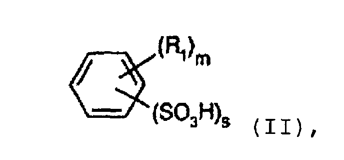 dans laquelle chaque R₁ est choisi indépendamment dans le groupe constitué par les alkyle en C₈-C₂₂ et les alkylaryle en C₈-C₂₂, m est un nombre compris entre 1 et 5, n est un nombre compris entre 1 et 3, et s est un nombre compris entre 1 et 3, avec au moins une base de type amine éthoxylée de formule IV : 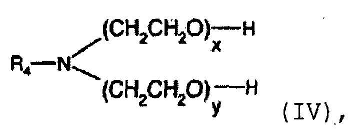 dans laquelle R₄ est choisi dans le groupe constitué par les alkyle en C₇-C₁₉ et les alcényle en C₇-C₁₉ -CH₂- et x + y est un nombre compris entre 2 et 15 inclus.

6. Procédé selon la revendication 5, **caractérisé en ce que** ledit un acide sulfonique aromatique est un composé défini par la formule IIa : 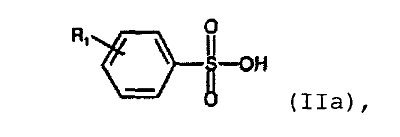 dans laquelle R₁ est un alkyle en C₈-C₂₂ ; et
ladite une base de type amine éthoxylée est représentée par la formule IV : 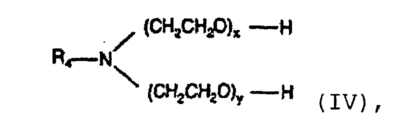 dans laquelle R₄ est un alkyle en C₇-C₁₉ et x + y est un nombre compris entre environ 7 et 9.

7. Procédé selon la revendication 5, **caractérisé en ce que** le rapport entre les composants II et IV c-à-d de l'acide sulfonique aromatique sur la base de type amine éthoxylée dans la solution d'isopropanol et d'eau soit régulé précisément de façon à ce que la gamme de pH, en tant que facteur de contrôle de la préparation du composé tensioactif ayant la formule [(H-B)⁺]ₙ Aⁿ⁻, corresponde à un pH compris entre 3 et 7.

8. Procédé selon la revendication 5, **caractérisé en ce que** le rapport entre les composants II et IV c-à-d de l'acide sulfonique aromatique sur la base de type amine éthoxylée dans la solution d'isopropanol et d'eau soit régulé précisément de façon à ce que la gamme de pH, en tant que facteur de contrôle de la préparation du composé tensioactif ayant la formule [(H-B)⁺]ₙ Aⁿ⁻, corresponde à un pH compris entre 4 et 6.

9. Procédé selon la revendication 5, **caractérisé en ce que** le rapport entre les composants II et IV c-à-d de l'acide sulfonique aromatique sur la base de type amine éthoxylée dans la solution d'isopropanol et d'eau soit régulé précisément de façon à ce que la gamme de pH, en tant que facteur de contrôle de la préparation du composé tensioactif ayant la formule [(H-B)⁺]ₙ Aⁿ⁻, corresponde à un pH compris entre 5 et 6.

10. Formulation pesticide selon la revendication 1 dans laquelle le un pesticide est choisi dans le groupe constitué par le mefenoxam, le s-metolachlore, le flumetsulam, le fluthiacet-méthyl, l'atrazine et le glyphosate.

11. Procédé de protection des cultures comprenant l'étape consistant à appliquer sur le lieu des cultures nécessitant une protection une quantité efficace sur le plan agricole de la formulation selon la revendication 1.

12. Procédé de protection des cultures comprenant l'étape consistant à appliquer sur le lieu des cultures nécessitant une protection une quantité efficace sur le plan agricole de la formulation selon la revendication 2.
